# EUROPÄISCHE PATENTSCHRIFT

(11) **EP 3 336 196 B1**
(45) Veröffentlichungstag und Bekanntmachung des Hinweises auf die Patenterteilung: **04.03.2020**
(21) Anmeldenummer: 16204599.1
(22) Anmeldetag: 16.12.2016
(51) Int. Cl.: C12Q 1/6895

(54) **WEIZENMARKER UND IHRE VERWENDUNG**
WHEAT MARKERS AND THEIR USE
MARQUEUR DE FROMENT ET SON UTILISATION

(43) Veröffentlichungstag der Anmeldung: 20.06.2018
(73) Patentinhaber: Strube Research GmbH & Co. KG, 38387 Söllingen (DE)
(72) Erfinder: Dr. Jacobi, Andreas, D-38364 Schöningen (DE)
(74) Vertreter: Helbig, Christian

(56) Entgegenhaltungen:
- WO-A1-2017/158127
- WO-A1-2017/158128
- WO-A1-2018/015403
- WO-A1-2018/015404
- CN-A- 1 769 448
- DATABASE EMBL [Online] 4. April 2006 (2006-04-04), "Triticum aestivum cDNA clone rwhdp13m06 3', Y.Ogihara unpublished cDNA library Wh_DPA20, mRNA sequence.", XP002768695, gefunden im EBI accession no. EM_EST:CJ524567 Database accession no. CJ524567
- DATABASE EMBL [Online] 15. Juli 2003 (2003-07-15), "AZO5.001A20F010110 AZO5 Triticum aestivum cDNA clone AZO5001A20, mRNA sequence.", XP002768696, gefunden im EBI accession no. EM_EST:CD880010 Database accession no. CD880010
- DATABASE EMBL [Online] 4. April 2006 (2006-04-04), "Triticum aestivum cDNA clone rwhdp13m06 3', Y.Ogihara unpublished cDNA library Wh_DPA20, mRNA sequence.", XP002768697, gefunden im EBI accession no. EM_EST:CJ524567 Database accession no. CJ524567
- DATABASE EMBL [Online] 15. Juli 2003 (2003-07-15), "G174.101I16F010821 G174 Triticum aestivum cDNA clone G174101I16, mRNA sequence.", XP002768698, gefunden im EBI accession no. EM_EST:CD895992 Database accession no. CD895992
- DATABASE EMBL [Online] 19. August 2012 (2012-08-19), "TSA: Triticum aestivum Ta_Contig47346.ansp mRNA sequence.", XP002768699, gefunden im EBI accession no. EM_TSA:JV940260 Database accession no. JV940260
- TALAAT AHMED ET AL: "QTL analysis of fertility-restoration against cytoplasmic male sterility in wheat.", GENES AND GENETIC SYSTEMS, Bd. 76, Nr. 1, 1. Januar 2001 (2001-01-01) , Seiten 33-38, XP055358955, JP ISSN: 1341-7568, DOI: 10.1266/ggs.76.33
- WENCHUN ZHOU ET AL: "SSR markers associated with fertility restoration genes against Triticum timopheevii cytoplasm in Triticum aestivum", EUPHYTICA, KLUWER ACADEMIC PUBLISHERS, DO, Bd. 141, Nr. 1-2, 1. Januar 2005 (2005-01-01), Seiten 33-40, XP019240826, ISSN: 1573-5060, DOI: 10.1007/S10681-005-5067-5
- TOSHIO KOJIMA ET AL: "High-resolution RFLP mapping of the fertility restoration (Rf3) gene against Triticum timopheevi cytoplasm located on chromosome 1BS of common wheat.", GENES AND GENETIC SYSTEMS, Bd. 72, Nr. 6, 23. August 1997 (1997-08-23), Seiten 353-359, XP055358980, JP ISSN: 1341-7568, DOI: 10.1266/ggs.72.353
- STOJALOWSKI STEFAN ET AL: "The importance of chromosomes from the sixth homeologic group in the restoration of male fertility in winter triticale with Triticum timopheevii cytoplasm", JOURNAL OF APPLIED GENETICS: AN INTERNATIONAL JOURNAL OF GENETICS AND BREEDING, SPRINGER, GERMANY, Bd. 54, Nr. 2, 29. März 2013 (2013-03-29), Seiten 179-184, XP035367195, ISSN: 1234-1983, DOI: 10.1007/S13353-013-0144-2 [gefunden am 2013-03-29]

### SEQUENCE LISTING

<110> STRUBE-RESEARCH GmbH & Co. KG
<120> Marker für Fertilitätswiederherstellung im Weizen
<130> ST20392EP
<140> ST20392EP
   <141> 2016-12-14
<160> 3
<170> PatentIn version 3.5
<210> 1
   <211> 101
   <212> DNA
   <213> Triticum aestivum
<400> 1
   cgacgacgac gaggatgccg agtttgatga catggaggat tatatcgacg rcgcggactg        60
   ggacgccgac atgtatgatg atgtgttcga tgtctgaagg a                           101
<210> 2
   <211> 201
   <212> DNA
   <213> Triticum aestivum
<400> 2
   tagttttctt cttggttgcc tgtaataaca tcataggttg aggttaccag ccgctccgca        60
   taaagcttga aagttgaact tgctgaagcg gcgagcctcg ygagcaccac caccaccgac       120
   aagtatcagg agcacctttc cctccagtac atgtaaataa aaaggagtat acatatttgt       180
   cctatgtatc gatttgagaa t                                                 201
<210> 3
   <211> 101
   <212> DNA
   <213> Triticum aestivum
<400> 3
   aactttatgc atgcgccatt gcagatcaat atctagtaga tcaatcagct kctctcgcct        60
   aacaggatca actccatcaa ctccaaatat catgtgctca g                           101

## Beschreibung

Die Erfindung betrifft Marker, deren Verfahren zum Screenen und/oder Selektion sowie die Verwendung der Marker hierzu.

### Hintergrund der Erfindung

Molekulare Marker sind Nachweismittel, um bestimmte Eigenschaften auf molekularer Ebene zu identifizieren oder/und deren Auftreten zu verfolgen.

Eine Vielzahl von Markern kann zum Screenen oder Selektieren von Eigenschaften in Pflanzen oder Pflanzenzellen genutzt werden.

Hierbei stellen SNPs (single nucleotide polymorphism) eine besondere Gruppe von Markern dar, die auch als genetische Marker bezeichnet werden.

Die Verbindung von SNPs zu einem bestimmten Gen und damit einer bestimmten Eigenschaft (oder Phänotyp) kann mit bekannten genetischen Mitteln bestimmt werden. Diese Verbindung oder das Auftreten von einem bestimmten mit einem Marker in Verbindung stehenden Merkmal nennt man auch Marker-Phänotyp Assoziation.

In Züchtungsverfahren will man bestimmte Eigenschaften einer Pflanze anreichern, d.h. man selektiert auf bestimmte Merkmale oder Eigenschaften der Pflanze. Zum Nachweis von Merkmalen bzw. der Wahrscheinlichkeit des Auftretens eines Merkmals mittels Markern kann nun ein SNP oder eine Gruppe von SNPs verwendet werden.

Ein wichtiges Merkmal ist der Ertrag einer Pflanze. Hierbei hat sich herausgestellt, dass Hybrid-Sorten vorteilhafte Eigenschaften wie Ertragstabilität zeigen. In Hybrid-Sorten werden genetische Pools mit unterschiedlichen Eigenschaften, die auf männliche und weibliche Kreuzungspartner verteilt sein können, durch deren Kreuzung kombiniert.

Ein Ansatz auf dem Gebiet der Hybrid-Pflanzenherstellung ist die Verwendung von CMS (cytoplasmatische männliche Sterilität) Pflanzen. Es wurden erfolgreich Hybrid-Sorten in einer Reihe von Pflanzen wie Tabak hergestellt, die dieses System verwenden. Hierbei sind sog. Restorer-Gene (Rf) von Bedeutung.

Bisher konnte sich die Hybridzüchtung in Weizen nicht vollständig durchsetzen, weil zum einen das System von Gametoziden abhängig ist, die nicht nur phytotoxisch sein können, sondern der richtige Zeitpunkt der Applikation solcher entscheidend ist. Zum anderen muss das System kostengünstig in der Produktion sein. In der Vergangenheit wurden CMS Linien eingesetzt, die bestmöglich steril waren. Allerdings war die Fertilitätswiederherstellung durch die Restorer-Linien problematisch, sodass Verluste in der Einkörnung zu unbefriedigender Saatgutproduktionsmenge führten. Ein befriedigendes System in Bezug auf die Fertilitätswiederherstellung mittels Restorer-Linien konnte bisher nicht bereitgestellt werden. Auch wäre es wünschenswert, wenn die Fertilitätswiederherstellung mittels Restorer-Linien möglichst unabhängig von Umwelteinflüssen erzielt werden könnte.

Zwar erlauben phänotypische Analysen im Feld bisher die Detektion von fertilen Genotypen. Allerdings kann derselbe Genotyp im nächsten Jahr Fertilitätsprobleme aufweisen. Das führt dazu, dass eine große Zahl an potentiellen Restorer-Linien über mehrere Jahre angebaut und getestet werden muss, um vollständig Fertilität-restaurierende Restorer- Linien zu finden.

Somit ist eine Marker-gestützte Selektion wünschenswert, da diese die Detektion von Genotypen mit einem vollständigen Fertilitätsrestaurationssystem nicht nur beschleunigen, sondern dieses auch effizienter gestalten würde.

In Weizen war es bisher nicht möglich wirtschaftlich interessante Hybrid-Sorten mit dem Fertilitätsrestaurationssystem bereit zu stellen.

Es war somit eine der vorliegenden Anmeldung zugrunde liegende Aufgabe Mittel oder/und Screening-Verfahren bereitzustellen, die eine Grundlage für die Herstellung von Hybrid-Weizenpflanzen mit guten Ausbeuten ermöglichen.

Eine weitere Aufgabe war die Bereitstellung von Markern, die in Screening-Verfahren bei Weizenpflanzen verwendet werden können.

Eine weitere Aufgabe war es ein Verfahren bereit zu stellen, mit dem es möglich ist die Fertilität in männlichen sterilen Pflanzen wieder herzustellen.

Im Folgenden sollen einige Begriffe näher definiert werden. Im Übrigen sind die verwendeten Begriffe im Sinne der Erfindung nach dem allgemeinen Verständnis des Fachmannes zu verstehen.

"QTL T, QTL B und QTL W" im Sinne der Erfindung sind die Markergene Tdurum_contig50667_306 (QTL T), BS00011202_51 (QTL B) bzw. wsnp_CAP12_c4924_2241879 (QTL W) wie auch in Tabelle 1 dargestellt.

"CMS" ist die cytoplasmatische männliche Sterilität, die durch nicht zusammenpassen des Cytoplasma und der Kerngene zustande kommt. Meist verursacht durch extrem verschiedenes Material, das gekreuzt wurde.

"R-Linie" oder "Restorer-Linie" besitzt mindestens ein Fertilitätswiederherstellungsgen, das bei einer Kreuzung mit einem männlichsterilen Genotyp die männliche Fertilität wiederherstellen kann. In diesem Zusammenhang wird auch von "Restorergen" gesprochen.

"Maintainer" erhält die Pollensterilität im Kerngenom. Enthält ein normales Cytoplasma, das Pollenfertilität aufrechterhält.

"cM" steht für centi Morgan. 1cM bedeutet so viel wie 1 Trennung von zwei Orten auf der DNS nach 100 Meiosen.

"BLAST" steht für "basic local alignment search tool" und wird für den Abgleich von Sequenzen zu einer Datenbank oder zu einer Referenzsequenz.

"Allel" bezeichnet eine Variante eines Locus auf einem Chromosom.

"molekulare Marker" oder "Marker" werden in der Genetik eingesetzt um bestimmte Allele im Genom zu "markieren". Dies kann dazu genutzt werden um eine Aussage über einen Allel gekoppelten Phänotyp treffen zu können.

"Cytoplasma" ist der Überbegriff für das zelluläre Innere, das feste und flüssige Kompartimente enthält.

"Chromosom" bezeichnet eine cis-gekoppelte Ansammlung von Genen und Markern.

"Hybride" wird in der Züchtung das Material bezeichnet, das aus der Kreuzung einer mütterlichen und einer väterlichen Komponente entsteht.

"QTL" steht für "quantitative trait locus". Das ist eine Chromosomen-Region, die eine signifikante Wahrscheinlichkeit aufweist, zur Ausprägung eines quantitativen phänotypischen Merkmals beizutragen.

"Genotyp" repräsentiert eine individuelle Allele- und Genkonstellation.

"Phänotyp" beschreibt die Ausprägung der Merkmale einer Pflanze.

"Gametozid" ist eine Chemikalie, die im Weizen eingesetzt werden kann, um die Entwicklung der männlichen Blütenteile zu verhindern und somit diese zu sterilisieren.

"Transkript" ist eine in RNS umgeschriebene Sequenz eines Gens, die in eine Aminosäurensequenz übersetzt wird, welche wiederum ein Protein kodiert.

"Heterozygot" ist eine Allelekonstellation, die Mutterallele und Vaterallele gemischt enthält.

"Selbstung" beschreibt eine Methode, bei der die ausgewählte Pflanze nur den eigenen Pollen zur Bestäubung angeboten bekommt.

"F1" ist die erste filiale Generation, bei der die Allele der Mutterpflanze als auch der Vaterpflanze jeweils zu 50% das Genom nach deren Kreuzung (Gametenverschmelzung) kodieren.

"F2" ist eine zweite filiale Generation, der die erste vorangeht (F1). Bei den Pflanzen wird die F1 geselbstet, was zu einer Aufspaltung führt. Nach der zweiten Mendelschen Regel spaltet sich sowohl der Genotyp als auch der Phänotyp auf.

"Rf" bedeutet "restoration of fertility" und wird in dieser Beschreibung als ein Locus für die Fertilitätswiederherstellung auf einem Chromosom verwendet.

"Chi-quadrat-Test" steht für eine statistische Methode mit der eine beobachtete Verteilung eines Merkmals mit einer vorausgesagten verglichen wird. Dabei wird ein Wert generiert, der zur Aussage über die Signifikanz der beobachteten Verteilung herangezogen wird.

"SNPChip" steht für ein Verfahren, bei dem kurze Sequenzen auf einem Trägerobjekt aufgetragen werden um Polymophismen, auf der DNS verteilt, zu detektieren.

"Polymorph" bedeutet, dass an einer Stelle im Genom zwei Varianten von Allelen vorkommen können. Z.B.: das A Allel kommt von der Mutter und das B Allel kommt von dem Vater. Die F1 Nachkommen aus dieser Kreuzung tragen in einer Pflanze auf einem der Chromosomen das Allel A und auf dem anderen das B Allel.

"CI" steht für confidence intervall und definiert das Vertrauensintervall der flankierenden Marker eines QTL.

"Genetische Karte" ist eine Zusammenfassung der errechneten Rekombinationshäufigkeiten zwischen den Markern, die in centi Morgan (cM) angegeben werden.

"Umwelteinflüsse", in diesem Kontext sind hauptsächlich die Witterungsbedingungen gemeint, welche die Sterilität verursachen können.

"Fertilitätsbestimmung" im Sinne der Erfindung ist der Nachweis inwiefern eine Pflanze fertil ist.

"Fertilitätswiederherstellung" ist der Vorgang, bei dem das/die Restorer-Gen/e dazu beiträgt/beitragen, dass die Nachkommen einer sterilen Pflanze fertil sind.

"Allellink (LD /linkage disequilibrium)" gekoppelte Abschnitte auf dem Chromosom, die zu einem nachweisbaren Anteil an die Nachkommen weitervererbt werden.

Die Erfindung betrifft in einem Aspekt ein Verfahren zum Nachweis der Fertilität oder/und der Fertilitätswiederherstellung oder/und von Restorergenen in einer Weizenpflanze oder Teilen einer Weizenpflanze, wobei einer der genetischen Marker QTL B (QTL B = SEQ ID NO. 3) oder QTL W (QTL W = SEQ ID NO. 2) verwendet wird, oder eine Kombination von zwei oder drei genetischen Markern verwendet wird, wobei das Markergen oder die Markergene ausgewählt werden aus der Gruppe bestehend aus QTL T (QTL T = SEQ ID NO: 1), QTL B (QTL B = SEQ ID NO. 3) und QTL W (QTL W = SEQ ID NO. 2), und durch Nachweis der Allele dieser Marker die Fertilität oder der Grad der Fertilität bestimmt wird.

In einem weiteren Aspekt ein Verfahren wie oben beschrieben, wobei eine Kombination die Marker QTL T (SEQ ID NO: 1), QTL B (SEQ ID NO. 3) und QTL W (SEQ ID NO. 2) oder eine Kombination der Marker QTL T (SEQ ID NO: 1) und QTL B (SEQ ID NO. 3), oder der Marker QTL T (SEQ ID NO: 1) und QTL W (SEQ ID NO. 2) verwendet wird.

Die Erfindung betrifft in einem weiteren Aspekt ein Verfahren zur Selektion einer Pflanze, die ein oder mehrere funktionelle Restorergene für eine zytoplasmatische männliche Sterilität (CMS) in Weizen aufweist, umfassend die Schritte:
a.Screenen einer Pflanzenpopulation oder einer Sammlung oder Gruppe von Pflanzen für einen Marker QTL B (QTL B = SEQ ID NO. 3) oder QTL W (QTL W = SEQ ID NO. 2) oder für mindestens zwei Marker, wobei die Markergene ausgewählt werden aus der Gruppe bestehend aus QTL T (QTL T = SEQ ID NO: 1), QTL B (QTL B = SEQ ID NO. 3) und QTL W (QTL W = SEQ ID NO. 2)
b.Nachweisen der Markernukleinsäuren,
c.Identifizieren der Pflanzen, die die Markernukleinsäuren aufweisen,
d.Selektion der Pflanzen, die die Markernukleinsäuren aufweisen.

In einem Aspekt betrifft die vorliegende Offenbarung ein oder mehrere Markergene oder eine Kombination davon, vorzugsweise mindesten 2 Markergene, wobei das Markergen mit einem oder mehreren Restorergenen assoziiert ist (Vertrauensintervall) innerhalb von 17 Mb auf Chromosom 1B oberer Arm, innerhalb von 5 Mb auf Chromosom 6A unterer Arm oder/und innerhalb von 1 Mb auf Chromosom 6A oberer Arm zur Verwendung in einem Verfahren zur Bestimmung der Fertilität oder/und der Fertilitätswiederherstellung oder/und von Restorergenen oder/und von ausgewählten Allellen oder einer Allellkombination in einer Weizenpflanze oder Teilen davon.Das oder die Markergene oder die Kombination von Markergenen gemäß der Erfindung kann ausgewählt sein aus der Gruppe bestehend aus den Markern QTL T, QTL B und QTL W.

In einem weiteren Aspekt betrifft die Offenbarung ein Verfahren zum Nachweis der Fertilität oder/und der Fertilitätswiederherstellung oder/und von Restorergenen in einer Weizenpflanze oder Teilen einer Weizenpflanze, wobei ein genetischer Marker oder eine Kombination von genetischen Markern verwendet wird ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm, das oder die Markergene vorzugsweise ausgewählt werden aus der Gruppe bestehend aus QTL T, QTL B und QTL W, und die Fertilität oder der Grad der Fertilität bestimmt wird.

In einem weiteren Aspekt betrifft die Offenbarung ein Verfahren zum Nachweis eines ausgewählten Allels oder einer ausgewählten Allelkombination in einer Weizenpflanze oder Teilen einer Weizenpflanze, wobei ein genetischer Marker oder eine Kombination von genetischen Markern ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm oder ausgewählt aus der Gruppe QTL T, QTL B und QTL W, verwendet werden und die ausgewählten Allele bestimmt werden.

In noch einem weiteren Aspekt betrifft die Offenbarung ein Verfahren zur Selektion einer Pflanze, die ein oder mehrere funktionelle Restorergene für eine zytoplasmatische männliche Sterilität (CMS) in Weizen aufweist, umfassend die Schritte:
e.Screenen einer Pflanzenpopulation oder einer Sammlung oder Gruppe von Pflanzen für mindestens einen oder zwei Marker, vorzugsweise drei Marker, wobei die Markernukleinsäure ein Allel umfasst, das einen Allellink (LD /linkage disequilibrium) mit einem oder mehreren Restorergenen darstellt oder sich auf einem oder mehreren Restorergenen befindet,
f.Nachweisen der Markernukleinsäuren,
g.Identifizieren der Pflanzen, die die Markernukleinsäuren aufweisen,
h.Selektion der Pflanzen, die die Markernukleinsäuren aufweisen.

Dieses Verfahren wie hierin offenbart kann die zusätzlichen Schritte umfassen: a. Erhalten der Pflanze, die ein oder mehrere Restorergene enthält, b. Kreuzen der Pflanze mit einer zweiten Pflanze, die ein oder mehrere Restorergene enthält, um Nachkommenpflanzen zu erhalten, c. Screenen der Nachkommenpflanzen für zwei oder drei der Marker aus der Gruppe, bei der das Markergen mit einem oder mehreren Restorergenen assoziiert ist (Vertrauensintervall) innerhalb von 17 Mb auf Chromosom 1B oberer Arm, innerhalb von 5 Mb auf Chromosom 6A unterer Arm oder/und innerhalb von 1 Mb auf Chromosom 6A oberer Arm oder für zwei oder drei der Marker QTL T, QTL B und QTL W, d. Selektion der Pflanzen, die eine oder mehrere Markernukleinsäuren ausgewählt von QTL T, QTL B und QTL W aufweist.

In diesem Verfahren wie hierin offenbart nach können die Schritte a.) bis d.) wiederholt werden, um eine Pflanze zu erhalten, die alle drei der Marker aus der Gruppe, bei der das Markergen mit einem oder mehreren Restorergenen assoziiert ist (Vertrauensintervall) innerhalb von 17 Mb auf Chromosom 1B oberer Arm, innerhalb von 5 Mb auf Chromosom 6A unterer Arm oder/und innerhalb von 1 Mb auf Chromosom 6A oberer Arm aufweist oder die alle drei der Marker QTL T, QTL B und QTL W aufweist (Pyramidisierung).

In noch einem weiteren Aspekt betrifft die Offenbarung die Verwendung von einem oder mehreren genetischen Markern wie oben beschrieben zum Screenen oder zur Selektion von Pflanzen oder/und zur Pyramidisierung.

In der erfindungsgemäßen Marker-Kombination oder dem erfindungsgemäßen Verfahren kann die Kombination die Marker QTL T und QTL B, oder QTL T und QTL W umfassen oder aus ihr bestehen bzw. das erfindungsgemäße Verfahren eine Kombination der Marker QTL T und QTL B, oder der Marker QTL T und QTL W oder der Marker QTL T, QTL B und QTL W verwenden.

Bei der erfindungsgemäßen Marker-Kombination oder dem erfindungsgemäßen Verfahren kann einer der Marker auf Chromosom 1B liegen.

Überraschender Weise konnten bereits mit einem der hier offenbarten Marker oder einer Kombination von zwei Markern gemäß der Erfindung Pflanzen identifiziert werden, die eine verbesserte Fertilität aufweisen. Vorteilhafter Weise konnten so für Hybrid-Sorten geeignete Pflanzen identifiziert werden.

Es war überraschend, dass die erfindungsgemäße Markerkombination dazu geeignet ist die Fertilität in einer Weizenpflanze zu einem hohen Prozentsatz korrekt zu bestimmen (Tabelle 2).

In 180 heterozygoten Testpflanzen für den Marker QTL T auf Chromosom 1B konnten beispielsweise 75% der fertilen Pflanzen mit dem Marker assoziiert werden. QTL T konnte im homozygoten Zustand mit dem Allele T 96% der vollfertilen detektieren (Tabelle 2). Zu einer Kombination von heterozygoten Marker QTL T und homozygoten Alleles für Fertilitätswiederherstellung des QTL W konnten bereits 95% der vollfertilen Genotypen assoziiert werden. Wenn der Marker QTL W durch den Marker QTL B ausgetauscht wurde, konnte 98% der Marker Allele Kombination mit dem Phänotyp assoziiert werden (Tabelle 4). Eine 100%-ge Fertilitätswiederherstellung konnte mit der Kombination der drei Marker beobachtet werden (Tabelle 5).

Die Erfindung stellt mit einer Allelekombination des fertilen Elters auf zwei unterschiedlichen Chromosomen ein neuartiges System dar, das in so einer Weise noch nirgendwo beschrieben wurde.

Ein weiterer Vorteil des erfindungsgemäßen Verfahrens und der erfindungsgemäßen Kombination von Markern ist, dass die Anreicherung der Fertilitätswiederherstellenden Allele mit diesen drei Markern überprüft werden kann.

Weitere Vorteile des erfindungsgemäßen Verfahrens und der erfindungsgemäßen Kombination von Markern ist auch, dass eine Detektion von sterilen CMS Linien mittels der drei molekularen Marker auch möglich ist, wenn es im spaltenden Restorermaterial auf die entsprechenden Allele selektiert wird (Tabelle 5). Weiterhin können auch Maintainer selektiert werden, wenn der Phänotyp eines Genotyps fertil ist, trotz der drei Allele für Sterilität (CCC) wie in Tabelle 5 aufgeführt. Das ist der Fall, wenn das Cytoplasma die Fertilität verursacht. Weiterhin von Vorteil ist, dass die Fertilitätswiederherstellung in homozygoter Form für die drei Marker nicht von Umweltfaktoren beeinflusst wird. Schließlich wird es mit dem erfindungsgemäßen Verfahren und Markerkombination möglich eine wirtschaftlich interessante Hybridproduktion mit diesem Systemansatz anzustreben.

Der erfindungsgemäße Markerkombinationseinsatz für die Fertilitätswiederherstellung erlaubt eine präzise Produktion von Restorer-Linien, die eine wirtschaftliche Produktion von Hybridsaatgut mit einer entsprechenden CMS-Linie rentabel machen kann. Im Übrigen sind die oben beschriebenen Merkmale in der technisch möglichen Art und Weise kombinierbar auch wenn dies in den Ausführungen oben und in den Beispielen nicht explizit beschrieben wurde.

Die Erfindung soll durch die folgenden Beispiele illustriert werden ohne dass diese als Einschränkung verstanden werden sollen.

### Beispiele

### 1. Pflanzen Material und Vorgehen zur Identifizierung der erfindungsgemäßen Marker

Der Elter, der als Mutter für die Kreuzung eingesetzt wurde, wurde kastriert (P1). Dieser Elter enthält das für die Sterilität induzierende Cytoplasma von Triticum Timopheevii. Des Weiteren enthält dieser Elter (P1) das/die entsprechende/n Rf Gen/e für die Fertilitätswiederherstellung.

Der P1 wurde mit einem Maintainer (P2 ohne Fertilitätswiederherstellende Gene) gekreuzt, um (I) die Rf Gene heterozygot in der F1 Hybride zu erhalten und (II) das steril induzierende Cytoplasma nicht zu wechseln. Denn das Cytoplasma wird immer von der Mutter an die Nachkommen weitervererbt.

Die Hybride (F1) wurde geselbstet (> 1 F1 Pflanzen eingesetzt), um eine phänotypische Aufspaltung in der F2 Generation für die Fertilitätswiederherstellung zu erhalten. Die F2 Population wurde im Feld dann angezogen.

### 2. Durchführung der Phänotypisierung

Die Bonitur erfolgte für vier F2 Populationen, die aus vier unterschiedlichen Elternpaarungen bestanden, wobei die vier Mütter auf den gleichen Ursprung für Fertilitätswiederherstellung zurückgehen.

Danach erfolgte eine Bonitur: vollfertil, teilfertil(∼50% fertil), spitzensteril (∼75% fertil), vollsteril.

Die phänotypischen Merkmale der Fertilitätswiederherstellung aller voll-, spitzen- und teilfertilen F2 Genotypen wurden zu einer Gruppe zusammengefasst ("fertil") und die vollsterilen Pflanzen zu der anderen Gruppe zugeordnet. Zwei von vier Populationen zeigten eine 3:1 Verteilung (fertil:steril), die andeutete, dass das/die Rf Gen/e eine dominante Wirkung auf die Fertilitätswiederherstellung hat/haben und dass ein Hauptgen mit einem großen phänotypischen Effekt an der Fertilitätswiederherstellung beteiligt ist. Von den beiden 3:1 verteilten Populationen wurde die anhand des chi-quadrat-Tests am besten verteilte für die Genotypisierung eingesetzt.

### 3. Genotypisierung und Erstellung der genetischen Karte

Die Genotypisierung von 346 F2 Genotypen und der entsprechenden Eltern erfolgte bei der Firma TraitGenetics (Gatersleben, Deutschland) mittels einem 15K SNPChip für Weizen.

Von den 13006 SNP-Markern konnten 3695 polymorphe zwischen den beiden Eltern der genotypisierten F2 Population extrahiert und für weitere Analysen eingesetzt werden. Die Zuordnung zu den Kopplungsgruppen/Chromosomen wurde für alle polymorphe Marker mittels BLAST und der aktuell verfügbaren physikalische Assemblierung (09.08.2016 Triticum aestivum.TGACv1.dna.toplevel.fa.gz) des Weizengenoms ermittelt (Gramene).

Marker, welche zu keinem Chromosom annotiert werden konnten, wurden manuell unter der Zuhilfenahme des Webtools https://triticeaetoolbox.org entsprechendem Chromosom zugewiesen. Marker ohne eine konkrete Chromosomzuweisung wurden von weiteren Analysen ausgeschlossen. Von den 3695 SNP-Markern waren 1694 für die genetische Kartenerstellung redundant. Mit 2001 SNP-Markern wurde eine genetische Karte für die 21 Weizen-Chromosome berechnet. Dafür wurde die freiverfügbare Software Carthagene (De Givry et al., 2004) benutzt.

### 4. QTL-Detektion

Die QTL Detektion erfolgte mit der Statistik-Software R unter dem Einsatz des R Packages R/qtl wie in Benke et al. (2014) beschrieben. Es wurden 3 signifikante QTLs detektiert: 1 auf Chromosom 1B und 2 auf Chromosom 6A (Tabelle 1).

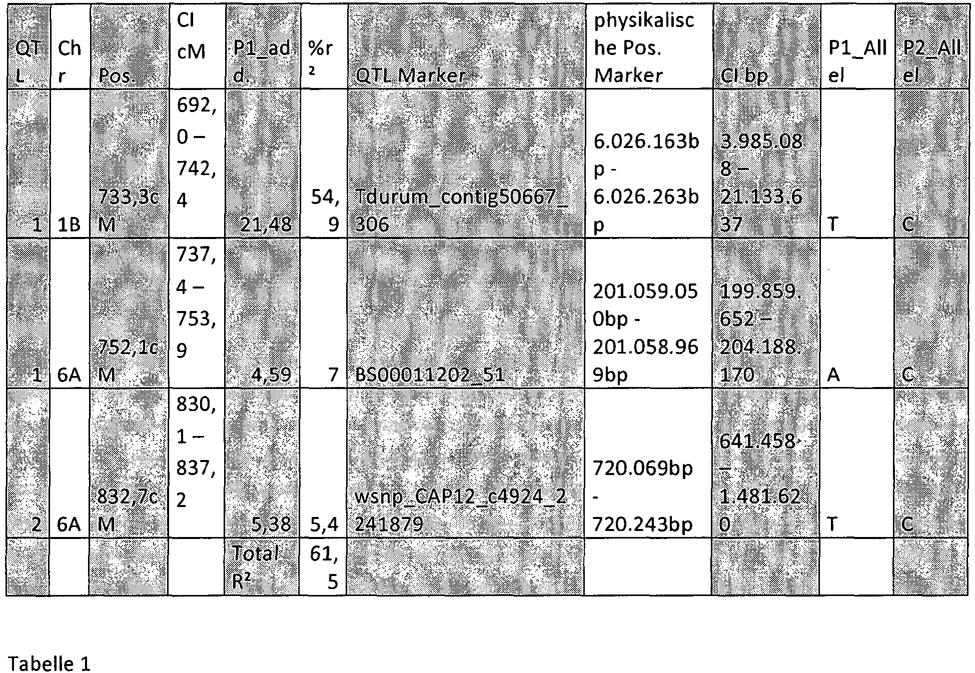

Von vorangegangenen publizierten Studien wurde bereits gezeigt, dass Chromosom 1B das Gen Rf3 beherbergt (Ahmed et al. 2001), welches als ein Hauptgen für die Fertilitätswiederherstellung dient. Allerdings wurden bisher weder die exakte physikalische Lokalisierung noch präzise Marker gezeigt. Genau dasselbe gilt für 6A, wo Rf6 lokalisiert wurde (Rongxia et al., 2002).

Erfindungsgemäß wurden 3 QTL und die entsprechenden Marker detektiert (Tabelle 1-5).

Der Einsatz von einem dieser Marker für die Fertilitätsvoraussage gibt bereits einen Hinweis beim Fertilitätsnachweis; eine verbesserte Aussage kann mit zwei der erfindungsgemäßen Marker gemacht werden. In Kombination zeigen zwei und besser alle drei erfindungsgemäßen Marker eine präzise Voraussage der Fertilität in der Genetik, wenn die Allele "T", "A", und "T" des P1 Elter zusammen auftreten. Es ist naheliegend, dass diese Allelekombination widerstandsfähiger gegenüber der Teil-, Spitzen- und Vollsterilität ist, die von Umwelteinflüssen verursacht wurde. Ein entsprechender Nachweis für die F3 Population kann auch durchgeführt werden.

Es konnte gezeigt werden, dass wenn der Marker auf Chromosom 1B homozygot für T oder heterozygot ist und die beiden Marker auf Chromosom 6A homozygot für T und A Allele sind, werden die Genotypen vollständig fertil sein (Tabelle 5).

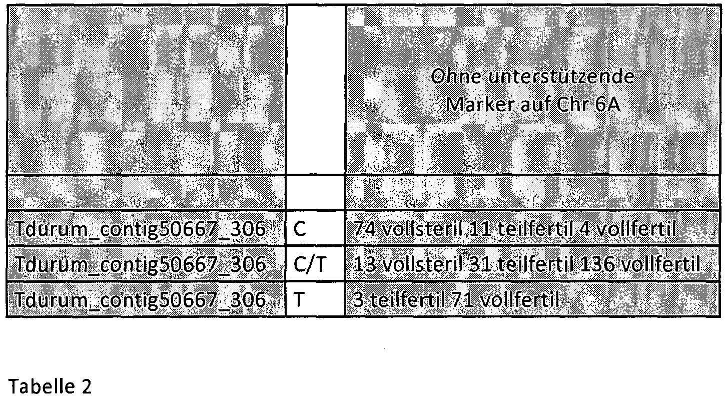

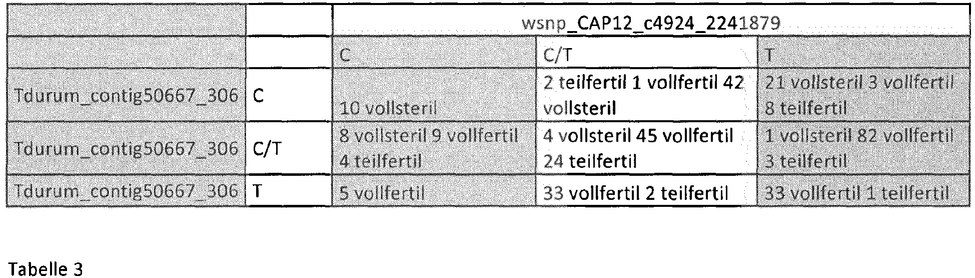

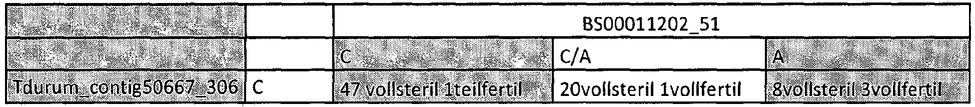

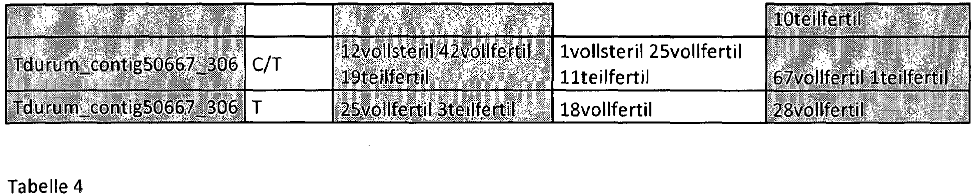

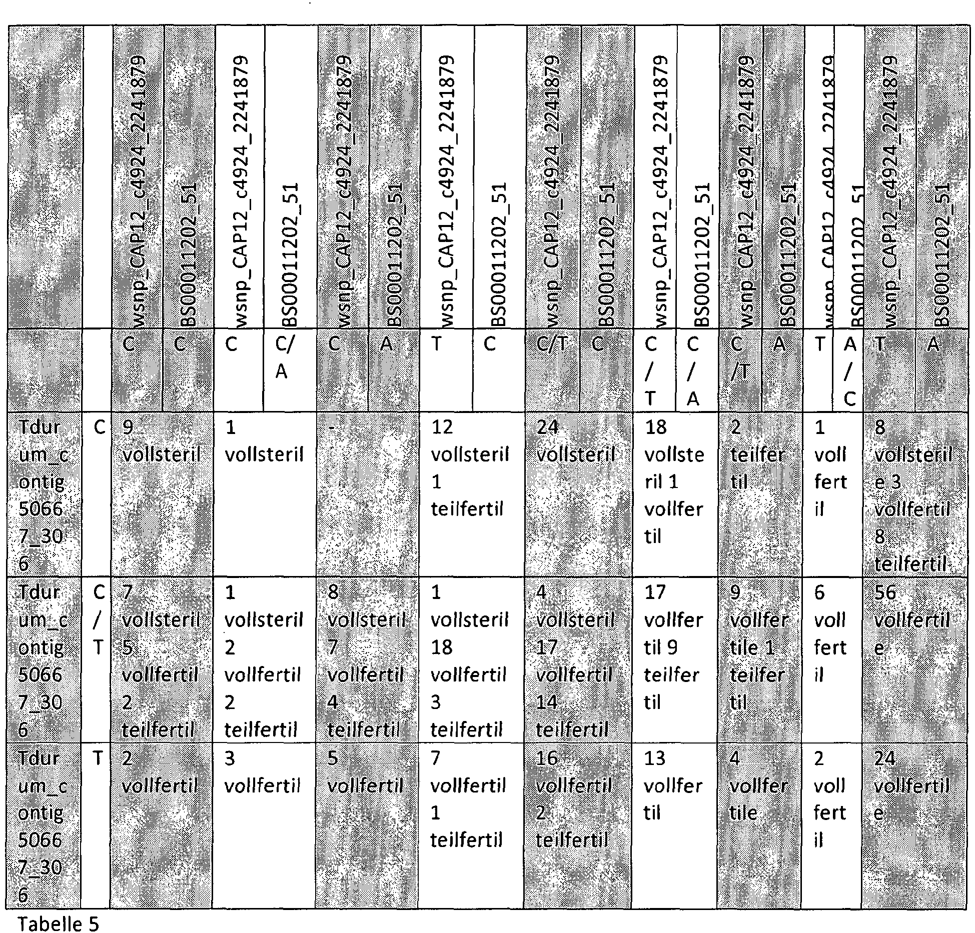

**Tabelle 6**

| Name | Chromosom | Sequenz |
|---|---|---|
| Tdurum_ contig50 667_306 QTL T SEQ ID NO.1 | 1B | |
| wsnp_C AP12_c4 924_224 1879 QTL W SEQ ID NO.2 | 6A | |
| BS00011 202_51 QTL B SEQ ID NO.3 | 6A | |

### Referenzen

Benke et al. 2014. The genetic basis of natural variation for iron homeostasis in the maize IBM population.
Barkan et al. 2012. A Combinatorial Amino Acid Code for RNA Recognition by Pentatricopeptide Repeat Proteins.
Uyttewaal et al. 2008. Characterization of Raphanus sativus Pentatricopeptide Repeat Proteins Encoded by the Fertility Restorer Locus for Ogura Cytoplasmic Male Sterility.
Ahmed et al. 2001. QTL analysis of fertility-restoration against cytoplasmic male sterility in wheat.
Kazama et al., 2008. Suppression mechanism of mitochondrial ORF79 accumulation by Rf1 protein in BT-type cytoplasmic male sterile rice.
Liu et al., 2016. A Mitochondria-Targeted PPR Protein Restores pol Cytoplasmic Male Sterility by Reducing orf224 Transcript Levels in Oilseed Rape
Whitford et al. 2013. Hybrid breeding in wheat: Technologies to improve hybrid wheat seed production
De Givry et al., 2004. Carhta Gene: multipopulation integrated genetic and radiation hybrid mapping.
Rongxia et al., 2002. Analysis of the fertility restoring gene Rf6 in T. timopheevii cytoplasmic male sterility of wheat with ISSR markers

## Patentansprüche

1. Verfahren zum Nachweis der Fertilität oder/und der Fertilitätswiederherstellung oder/und von Restorergenen in einer Weizenpflanze oder Teilen einer Weizenpflanze, wobei einer der genetischen Marker QTL B (QTL B = SEQ ID NO. 3) oder QTL W (QTL W = SEQ ID NO. 2) verwendet wird, oder eine Kombination von zwei oder drei genetischen Markern verwendet wird, wobei das Markergen oder die Markergene ausgewählt werden aus der Gruppe bestehend aus QTL T (QTL T = SEQ ID NO: 1), QTL B (QTL B = SEQ ID NO. 3) und QTL W (QTL W = SEQ ID NO. 2), und durch Nachweis der Allele dieser Marker die Fertilität oder der Grad der Fertilität bestimmt wird.

2. Verfahren nach Anspruch 1, wobei eine Kombination die Marker QTL T (SEQ ID NO: 1), QTL B (SEQ ID NO. 3) und QTL W (SEQ ID NO. 2) oder eine Kombination der Marker QTL T (SEQ ID NO: 1) und QTL B (SEQ ID NO. 3), oder der Marker QTL T (SEQ ID NO: 1) und QTL W (SEQ ID NO. 2) verwendet wird.

3. Verfahren zur Selektion einer Pflanze, die ein oder mehrere funktionelle Restorergene für eine zytoplasmatische männliche Sterilität (CMS) in Weizen aufweist, umfassend die Schritte:
a. Screenen einer Pflanzenpopulation oder einer Sammlung oder Gruppe von Pflanzen für einen Marker QTL B (QTL B = SEQ ID NO. 3) oder QTL W (QTL W = SEQ ID NO. 2) oder für mindestens zwei Marker, wobei die Markergene ausgewählt werden aus der Gruppe bestehend aus QTL T (QTL T = SEQ ID NO: 1), QTL B (QTL B = SEQ ID NO. 3) und QTL W (QTL W = SEQ ID NO. 2)
b. Nachweisen der Markernukleinsäuren,
c. Identifizieren der Pflanzen, die die Markernukleinsäuren aufweisen,
d. Selektion der Pflanzen, die die Markernukleinsäuren aufweisen.

## Claims

1. Method for detecting the fertility or/and fertility restoration or/and restorer genes in a wheat plant or parts of a wheat plant, wherein one of the genetic markers QTL B (QTL B = SEQ ID NO: 3) or QTL W (QTL W = SEQ ID NO: 2) is used, or a combination of two or three genetic markers is used, wherein the marker gene or genes are selected from the group consisting of QTL T (QTL T = SEQ ID NO: 1), QTL B (QTL B = SEQ ID NO: 3) and QTL W (QTL W = SEQ ID NO: 2), and fertility or the degree of fertility is determined by detecting the alleles of these markers.

2. The method according to claim 1, wherein a combination of the markers QTL T (SEQ ID NO: 1), QTL B (SEQ ID NO: 3) and QTL W (SEQ ID NO: 2) or a combination of the markers QTL T (SEQ ID NO: 1) and QTL B (SEQ ID NO: 3) or the markers QTL T (SEQ ID NO: 1) and QTL W (SEQ ID NO: 2) is used.

3. A method of selecting a plant having one or more functional restorer genes for cytoplasmic male sterility (CMS) in wheat, comprising the steps of:
a. screening a plant population or a collection or group of plants for a marker QTL B (QTL B = SEQ ID NO: 3) or QTL W (QTL W = SEQ ID NO: 2) or for at least two markers, wherein the marker genes are selected from the group consisting of QTL T (QTL T = SEQ ID NO: 1), QTL B (QTL B = SEQ ID NO: 3) and QTL W (QTL W = SEQ ID NO: 2),
b. detecting the marker nucleic acids,
c. identifying the plants containing the marker nucleic acids,
d. selecting the plants containing the marker nucleic acids.

## Revendications

1. Procédé de détection de fertilité ou/et de restauration de fertilité ou/et de gènes de restauration dans une plante de blé ou des parties d'une plante de blé, dans lequel on utilise l'un des marqueurs génétiques QTL B (QTL B = SEQ ID NO : 3) ou QTL W (QTL W = SEQ ID NO : 2) ou une combinaison de deux ou trois marqueurs génétiques, le ou les gènes marqueurs étant choisis dans le groupe constitué de QTL T (QTL T = SEQ ID NO : 1), QTL B (QTL B = SEQ ID NO : 3) et QTL W (QTL W = SEQ ID NO : 2), la fertilité ou le degré de fertilité étant déterminés en détectant les allèles de ces marqueurs.

2. Procédé selon la revendication 1, dans lequel on utilise une combinaison des marqueurs QTL T (SEQ ID NO : 1), QTL B (SEQ ID NO : 3) et QTL W (SEQ ID NO : 2) ou une combinaison des marqueurs QTL T (SEQ ID NO : 1) et QTL B (SEQ ID NO : 3) ou des marqueurs QTL T (SEQ ID NO : 1) et QTL W (SEQ ID NO : 2).

3. Procédé de sélection d'une plante ayant un ou plusieurs gènes restaurateurs fonctionnels pour la stérilité mâle cytoplasmique (CMS) dans le blé comprenant les étapes consistant à :
a. cribler une population végétale ou une collection ou un groupe de plantes pour un marqueur QTL B (QTL B = SEQ ID NO : 3) ou QTL W (QTL W = SEQ ID NO : 2) ou pour au moins deux marqueurs, les gènes marqueurs étant choisis dans le groupe constitué par QTL T (QTL T = SEQ ID NO : 1), QTL B (QTL B = SEQ ID NO : 3) et QTLW (QTL W = SEQ ID NO : 2),
b. détecter les acides nucléiques marqueurs,
c. identifier les plantes contenant les acides nucléiques marqueurs,
d. sélectionner les plantes contenant les acides nucléiques marqueurs.
